# EUROPEAN PATENT APPLICATION

(11) **EP 4 696 768 A1**
(43) Date of publication of application: **18.02.2026**
(21) Application number: 24788766.4
(22) Date of filing: 10.04.2024
(51) Int. Cl.: C12M 1/34, C12M 3/00, C12N 5/077

(54) **DEVICE FOR DETECTING CELL CHARACTERISTIC AND CELL CHARACTERISTIC DETECTION SET**

(30) Priority: 11.04.2023 JP 2023064450; 18.12.2023 JP 2023212899
(71) Applicant: Sumitomo Bakelite Co.Ltd., Shinagawa-ku Tokyo 140-0002 (JP); Kyoto University, Kyoto-shi, Kyoto 606-8501 (JP)
(72) Inventor: TANAKA Hayao, Tokyo 140-0002 (JP); FUNAOKA Sohei, Tokyo 140-0002 (JP); YOSHIKUNI Takuro, Tokyo 140-0002 (JP); SAKURA Takeshi, Tokyo 140-0002 (JP); YOSHIDA Yoshinori, Kyoto-shi, Kyoto 606-8501 (JP); FUJIWARA Yuya, Kyoto-shi, Kyoto 606-8501 (JP); INAZUKA Fumika, Kyoto-shi, Kyoto 606-8501 (JP)
(74) Representative: Müller-Boré & Partner Patentanwälte PartG mbB
(86) International application number: PCT/JP2024/014556
(87) International publication number: WO 2024/214742

(57) **Abstract**

A cell characteristic detection device (10) includes: a base plate (20); and a pair of holders (30) made of resin, suspended from the base plate (20), and elastically deformable. The cell characteristic detection device (10) is configured to allow an external device to detect a displacement amount ΔW of at least one of the pair of holders (30) while the pair of holders (30) are holding a cell aggregate (15) therebetween.

## Description

### Technical Field

The present invention relates to a cell characteristic detection device and a cell characteristic detection set.

### Background Art

There has been known a cell characteristic detection device for detecting a cell characteristic. For example, Patent Literature 1 discloses a cell characteristic detection device that can detect the contraction characteristic of muscle cells retained on a support mainly made of collagen and having a long portion, by a strain gauge coupled to a coupler provided for one end of the long portion.

### Citation List

### Patent Literature

Patent Literature 1: Japanese Unexamined Patent Application Publication No. 2011-030574

### Summary of Invention

### Technical Problem

In the cell characteristic detection device of Patent Literature 1, the support for retaining cells is mainly made of collagen. Besides, the support is used in such a manner that the whole support is immersed in liquid containing a compound. This causes such a problem that non-specific adsorption of the compound easily occurs.

In view of this, it is desired to achieve a cell characteristic detection device that can hardly cause non-specific adsorption of a compound.

### Solution to Problem

A cell characteristic detection device according to the present invention includes: a base plate; and a pair of holders made of resin, suspended from the base plate, and elastically deformable. The cell characteristic detection device is configured to allow an external device to detect an amount of displacement of at least one of the holders while the pair of holders are holding a cell aggregate therebetween.

With this configuration, only distal end portions of the holders suspended in the cell characteristic detection device can be immersed in liquid. Besides, the holders are made of resin, and therefore, non-specific adsorption of a compound is hard to occur from the viewpoint of a contact area and a material.

A cell characteristic detection set according to the present invention includes the cell characteristic detection device, and a container having an inner surface subjected to a cell non-specific adsorption inhibition treatment.

With this configuration, since the container having the inner surface subjected to the cell non-specific adsorption inhibition treatment is used, adsorption of a cell aggregate to the cell characteristic detection device is secured, and non-specific adsorption of cells to the container is hard to occur.

The following describes preferred aspects of the present invention. However, the scope of the present invention is not limited by the preferred aspects described below.

As one aspect, in the cell characteristic detection device, the pair of holders may be a pair of film bodies facing each other.

With this configuration, it is possible to easily change a resolving power for measurement of the displacement amount and a measurement maximum displacement amount by changing the thickness of the pair of film bodies.

As one aspect, in the cell characteristic detection device, the pair of film bodies may be made of a resin material having a Young's modulus of 100 MPa or more but 4500 MPa or less and each have a thickness of 5 µm or more but 400 µm or less.

With this configuration, the resolving power for measurement of the displacement amount and the measurement maximum displacement amount can have more appropriate values.

As one aspect, in the cell characteristic detection device, the pair of holders may each include a holder body extending downward from the base plate, and a bent end in a form of a bend at a lower end of the holder body.

With this configuration, it is possible to stably retain a cell aggregate by the bent end.

As one aspect, in the cell characteristic detection device, the pair of holders may be made of polystyrene-based resin, polypropylene-based resin, or polyethylene-based resin.

With this configuration, non-specific adsorption of a compound to the cell characteristic detection device is harder to occur.

As one aspect, in the cell characteristic detection device, the cell aggregate may be a skeletal muscle cell aggregate, a cardiac muscle cell aggregate, or a smooth muscle cell aggregate.

With this configuration, with the use of the cell characteristic detection device that can hardly cause non-specific adsorption of a compound, it is possible to detect muscular contraction characteristics of the skeletal muscle cell aggregate, the cardiac muscle cell aggregate, or the smooth muscle cell aggregate more accurately.

As one aspect, the cell characteristic detection device may further include at least one contact member provided between the pair of holders and toward the base plate, the at least one contact member being configured to come into contact with the pair of holders from inside in response to contraction of the cell aggregate.

With this configuration, since the pair of holders deforming by the contraction of a cell aggregate abut with the contact member, the contact member gives a resistance to the cell aggregate to contract. As a result, the cell characteristic detection device strongly grows the cell aggregate.

As one aspect, in the cell characteristic detection device, the at least one contact member may include a plurality of contact members extending from the base plate over respective distances different from each other, the plurality of contact members being each attachable to and detachable from the base plate.

With this configuration, since a resistance given by the contact member to the cell aggregate to contract varies depending on the length of the contact member, a resistance corresponding to the cultural situation of the cell aggregate is given to the cell aggregate to contract.

Further features and advantages of the present invention will become clearer by the following illustrative and nonlimiting description of embodiments to be described with reference to the drawings.

### Brief Description of Drawings

Fig. 1 is a perspective view of a cell characteristic detection device of a cell characteristic detection set according to a first embodiment;
Fig. 2 is a perspective view of a container of the cell characteristic detection set according to the first embodiment;
Fig. 3 is a schematic view of the cell characteristic detection set and a cell aggregate according to the first embodiment;
Fig. 4 is a perspective view of a holder according to a second embodiment;
Fig. 5 is a sectional view of a cell characteristic detection set according to a third embodiment and illustrates a contact member attached to a base plate;
Fig. 6 is a sectional view of the cell characteristic detection set according to the third embodiment and illustrates a contact member attached to the base plate, the contact member being longer than the contact member illustrated in Fig. 5 in length from the base plate; and
Fig. 7 is a perspective view of a holder according to another embodiment.

### Description of Embodiments

### 1. First Embodiment

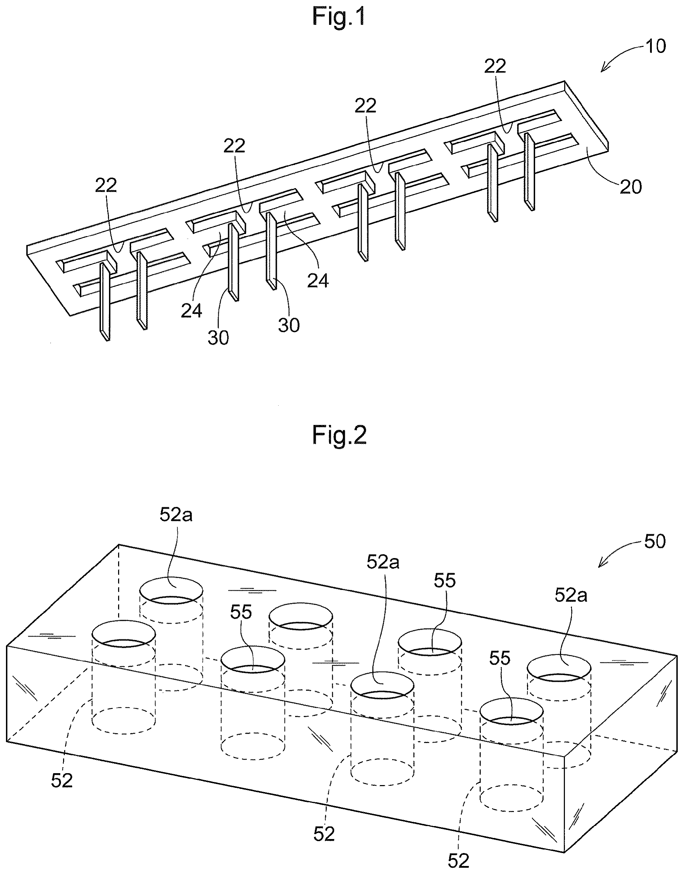

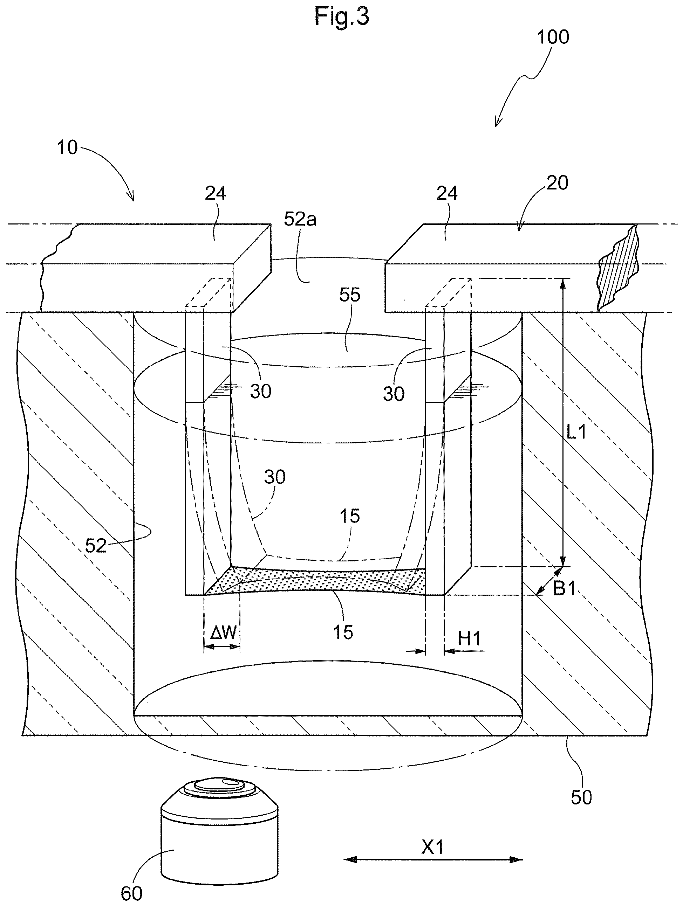

The following describes a cell characteristic detection set 100 according to a first embodiment, with reference to the drawings. The cell characteristic detection set 100 includes a cell characteristic detection device 10 and a container 50. Fig. 1 is a perspective view of the cell characteristic detection device 10, Fig. 2 is a perspective view of the container 50, and Fig. 3 is a schematic view of the cell characteristic detection set 100 and a cell aggregate 15. The cell characteristic detection device 10 for detecting a characteristic of cells, more specifically, a characteristic of the cell aggregate 15, includes a base plate 20, and a pair of holders 30 made of resin and suspended from the base plate 20 in such a manner as to be elastically deformable. The cell characteristic detection device 10 can detect a displacement amount of the holder 30, e.g., a displacement amount ΔW illustrated in Fig. 3 from outside with the cell aggregate 15 being retained between the pair of holders 30. Note that the cell aggregate indicates a cluster of cells, which cluster is formed by a plurality of cells being aggregated.

In the present embodiment, the base plate 20 is an oblong plate material made of resin and has four rectangular through-holes 22 formed in a thickness direction of the base plate 20 and arranged in a longitudinal direction thereof. The opposite side surfaces of the through-hole 22 of the base plate 20 in its longitudinal direction include respective bottom plate portions 24 extending toward the center of the through-hole 22 and held in a cantilever state. The bottom plate portion 24 includes the holder 30 suspended downward. As illustrated in Fig. 1, in the present embodiment, respective holders 30 suspended from the bottom plate portions 24 provided for the opposite side surfaces of one through-hole 22 make a pair. In the present embodiment, four pairs of holders 30 are provided. Here, "suspended" means to be provided in a suspended manner. A pair of holders 30 is provided to be suspended vertically in the example of Fig. 1, but the present invention is not limited to this. For example, a pair of holders 30 may be provided to be inclined from a vertical direction by an angle from ±10 degrees to ±20 degrees or the like.

The holder 30 may be integrated with the base plate 20 or may be detachably provided for the bottom plate portion 24 of the base plate 20. Here, a direction X1 indicates a direction where a pair of holders 30 is arranged, as illustrated in Fig. 3. In the present embodiment, the longitudinal direction of the base plate 20 is the same as the direction X1. The pair of holders 30 is preferably made of polystyrene-based resin, polypropylene-based resin, or polyethylene-based resin.

As illustrated in Fig. 2, the container 50 includes hollow portions 52 to be filled with liquid 55. The container 50 in the present embodiment is a casting container made of plastic, metal, glass, or the like. The casting container is a container for causing the device to retain a cell aggregate 15 and is a container in which cells are aggregated to form an aggregate. The container 50 includes a plurality of hollow portions 52. The number of hollow portions 52 is preferably a multiple of the number of pairs (in the present embodiment, "four") of holders 30 per cell characteristic detection device 10, and in the present embodiment, eight hollow portions 52 are provided. In this case, two cell characteristic detection devices 10 are attached to one container 50.

Fig. 3 is a view illustrating detection of the displacement amount ΔW of the holder 30, that is, measurement of the displacement amount of the cell aggregate 15. In Fig. 3, the cell aggregate 15 and the holders 30 bent on their distal end side due to contraction of the cell aggregate 15 are indicated by alternate long and two short dashes line. The cell aggregate 15 is a skeletal muscle cell aggregate, a cardiac muscle cell aggregate, or a smooth muscle cell aggregate, which is manufactured by inducing differentiation of induced pluripotent stem cells, for example. Preferably, the cell characteristic detection device 10 is also used to culture the cell aggregate 15. More preferably, the cell characteristic detection device 10 is also used for induced differentiation and maturation of the cell aggregate 15. For example, a cell aggregate 15 before induced differentiation is attached to respective distal ends of a pair of holders 30 of the cell characteristic detection device 10. Subsequently, the distal ends of the pair of holders 30 and the cell aggregate 15 before induced differentiation are immersed in a liquid culture medium in a culture vessel (not illustrated), and differentiation of the cell aggregate 15 is induced while the cell aggregate 15 is bridged between the pair of holder 30, so that a target cell aggregate 15, for example, a cardiac muscle cell aggregate may be retained between the pair of holders 30.

In the present embodiment, the pair of holders 30 is a pair of film bodies facing each other. As illustrated in Fig. 3, the pair of holders 30 as film bodies each have a film shape having a width B1, a thickness H1, and a length L1 from the bottom plate portion 24 to the distal end of the holder 30. The pair of holders 30 extend vertically from the base plate 20 so that a thickness direction thereof becomes the same direction as the direction X1, and the pair of holders 30 are elastically deformable in the direction X1 to connect the pair of holders 30, in response to their distal end sides (lower end sides) being bent in the thickness direction. Hereby, in response to contraction of the cell aggregate 15 retained by the pair of holders 30, the pair of holders 30 are elastically deformed in a direction where the distal ends of the pair of holders 30 approach each other, that is, in the direction X1. Here, the displacement amount of the cell aggregate 15 in the direction X1 is twice as large as ΔW, where ΔW is the displacement amount of one holder 30 in the direction X1.

When a maximum displacement amount by which the holder 30 can elastically deform is defined as a measurement maximum displacement amount ΔWmax, the measurement maximum displacement amount ΔWmax and the resolving power are changeable by changing the thickness H1, without changing a measurement apparatus for detecting the displacement amount ΔW, a resin material of the holder 30, and the length L1 and the width B1 of the holder 30. The pair of holders 30 as film bodies are preferably made of a resin material having a Young's modulus E of 100 MPa or more but 4500 MPa or less. More preferably, the pair of holders 30 are made of a resin material having a Young's modulus E of 300 MPa or more but 4300 MPa or less. Furthermore preferably, the pair of holders 30 are made of a resin material having a Young's modulus E of 400 MPa or more but 4000 MPa or less. Each of the pair of holders 30 preferably have a thickness H1 of 5 µm or more but 400 µm or less. More preferably, each of the pair of holders 30 have a thickness H1 of 7 µm or more but 200 µm or less. Furthermore preferably, each of the pair of holders 30 have a thickness H1 of 8 µm or more but 100 µm or less. Note that the Young's modulus E in the present specification is a measured value at 25°C (under room temperature).

In a case where the pair of holders 30 as film bodies are made of polystyrene-based resin, it is desirable that the Young's modulus E be 3000 MPa or more but 4000 MPa or less and the thickness H1 be 40 µm or more but 55 µm or less. In a case where the pair of holders 30 as film bodies are made of polypropylene-based resin, it is desirable that the Young's modulus E be 1500 MPa or more but 2500 MPa or less and the thickness H1 be 50 µm or more but 65 µm or less. As specific examples of the pair of holders 30 as film bodies, Table 1 exhibits the Young's modulus E, the measurement maximum displacement amount ΔWmax, the length L1, the width B1, and the thickness H1, in terms of Example 1 in which the holder 30 is made of polystyrene and Example 2 in which the holder 30 is made of polypropylene.

**[Table 1]**

| | Young's modulus E (MPa) | Measurement maximum displacement amount ΔWmax (mm) | Length L1 (mm) | Width B1 (mm) | Thickness H1 (mm) |
|---|---|---|---|---|---|
| Example 1 | 3500 | 0.71 | 12 | 2.5 | 0.048 |
| Example 2 | 2000 | 0.708 | 12 | 2.5 | 0.058 |

The resolving power can be changed by changing the measuring apparatus for detecting the displacement amount ΔW. In the cell characteristic detection set 100 according to the present embodiment, the container 50 is transparent or semitransparent, and an optical microscope 60 that can measure the displacement amount ΔW of the holder 30 is provided below the container 50. The optical microscope 60 corresponds to the measuring apparatus for detecting the displacement amount ΔW. The container 50 is filled with transparent or semitransparent liquid 55. Preferably, a lighting device is provided above the container 50. The liquid 55 is liquid containing a low molecular weight compound, for example. When the displacement amount ΔW of the holder 30 is measured and acquired as tension information, it is possible to evaluate the influence (toxicity) of the compound on the cardiac muscle cell aggregate, evaluate efficacy of the compound to a disease model of the cardiac muscle cell aggregate, and search for the most suitable compound for induced differentiation into the cardiac muscle cell aggregate.

Referring back to Fig. 2, a cell non-specific adsorption inhibition treatment is performed on an inner surface 52a of the hollow portion 52 in the container 50. The cell non-specific adsorption inhibition treatment is a treatment to inhibit non-specific adsorption of cells. The cell non-specific adsorption inhibition treatment can be performed by a hydrophilic treatment, for example. More specifically, the cell non-specific adsorption inhibition treatment can be performed by causing the inner surface 52a of the hollow portion 52 to contain at least one group selected from the group consisting of groups expressed as Formula (1), Formula (2), Formula (3), and Formula (4) as follows.

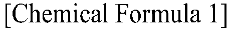

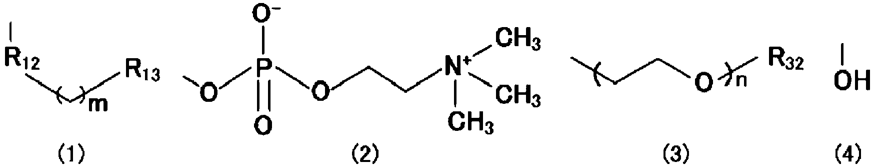

Here, in Formula (1), R₁₂ represents NH or an oxygen atom, and m represents an integer of 0 to 4. R₁₃ represents a hydrogen atom, a hydroxyl group, or a methoxy group. In Formula (3), R₃₂ represents a hydrogen atom or a methyl group, and n represents an integer of 2 to 100.

The cell non-specific adsorption inhibition treatment can be performed by forming, on the hollow portion 52, a coating layer mainly containing a polymer including a particular hydrophilic structural unit. Here, the hydrophilic structural unit in the polymer mainly contained in the coating layer can include at least one structural unit selected from the group consisting of structural units expressed as Formula (5), Formula (6), Formula (7), and Formula (8) as follows.

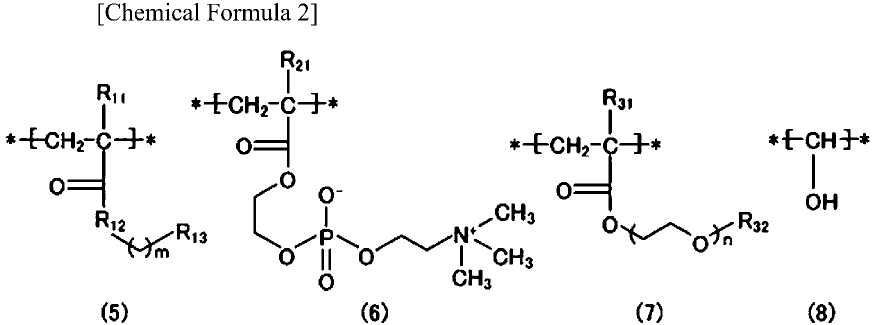

Here, * represents a combination. In Formula (5), R₁₁ represents a hydrogen atom or a methyl group. R₁₂ represents NH or an oxygen atom, and m represents an integer of 0 to 4. R₁₃ represents a hydrogen atom, a hydroxyl group, or a methoxy group. In Formula (6), R₂₁ represents a hydrogen atom or a methyl group. In Formula (7), R₃₁ represents a hydrogen atom or a methyl group. R₃₂ represents a hydrogen atom or a methyl group, and n represents an integer of 2 to 100.

As an example, in terms of the structural unit expressed as Formula (5), a monomer as a raw material of the hydrophilic structural unit in a case where R₁₁ is a hydrogen atom can be N-(2-hydroxyethyl) acrylic amide (HEAA), for example. A monomer as a raw material of the hydrophilic structural unit in a case where R₁₁ is a methyl group can be 2-hydroxyethyl methacrylate (HEMA), for example.

The hydrophilic structural unit in the polymer mainly contained in the coating layer may include a structural unit expressed as Formula (9) as follows.

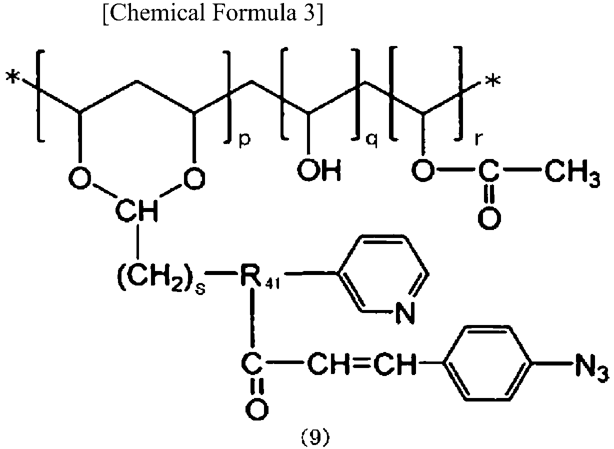

Here, * represents a combination. In Formula (9), R₄₁ represents an alkyl group having a carbonyl group and an amino group, p represents an integer of 1 to 1000, q represents an integer of 40 to 4995, r represents an integer of 0 to 4000, and s represents an integer of 1 to 3.

The polymer mainly contained in the coating layer may further include a hydrophobic structural unit. The hydrophobic structural unit in this case can include at least one structural unit selected from the group consisting of structural units expressed as Formula (10) and Formula (11) as follows.

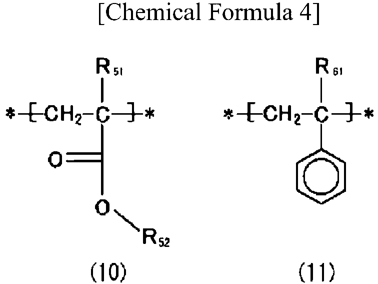

Here, * represents a combination. In Formula (10), R₅₁ represents a hydrogen atom or a methyl group. R₅₂ represents a straight or branched alkyl group with a carbon number of 1 to 10, an alicyclic alkyl group with a carbon number of 3 to 8, or a combination of them. In Formula (11), R₆₁ represents a hydrogen atom or a methyl group.

As an example, the straight or branched alkyl group with a carbon number of 1 to 10 can be a methyl group, an ethyl group, a propyl group, a methyl ethyl group, a butyl group, a 1,2-dimethyl ethyl group, a pentyl group, a 1-methyl butyl group, a 2-methyl butyl group, a hexyl group, and the like. The alicyclic alkyl group with a carbon number of 3 to 8 can be a cyclopropyl group, a cyclobutyl group, a cyclopentyl group, a cyclohexyl group, and the like.

It is preferable that a total content of the hydrophilic structural unit in the polymer mainly contained in the coating layer be 10 mol% or more. The total content of the hydrophilic structural unit is preferably 20 mol% or more but 80 mol% or less, and further preferably 30 mol% or more but 70 mol% or less. It is preferable that a total content of the hydrophobic structural unit in the polymer be 90 mol% or less. The total content of the hydrophobic structural unit is preferably 40 mol% or more but 80 mol% or less, and further preferably 50 mol% or more but 70 mol% or less.

The polymer mainly contained in the coating layer may further include a cross-linking structural unit in addition to the hydrophilic structural unit and the hydrophobic structural unit. The content of the cross-linking structural unit may be 0.05 mol% or more but 20 mol% or less, and preferably 0.5 mol% or more but 10 mol% or less, for example.

Due to such a coating layer, the inner surface 52a of the hollow portion 52 has contact with pure water at 25°C (under room temperature) by a contact angle of 3° or more but 90° or less. By hydrophilizing the inner surface 52a of the hollow portion 52, it is possible to inhibit a compound from nonspecifically adhering to the inner surface 52a of the hollow portion 52. Note that a treatment to make the contact angle less than 3° may be large-scaled and therefore unfavorable in terms of cost. In the meantime, if the contact angle is more than 90°, which is excessively large, the hydrophilic degree decreases, so that the inhibition ability to inhibit non-specific adsorption of a compound might decrease. When the contact angle is 3° or more but 90° or less, it is possible to inhibit non-specific adsorption of the compound to the inner surface 52a of the hollow portion 52 at a relatively low cost.

The contact angle of the inner surface 52a of the hollow portion 52 is preferably 5° or more, and more preferably 8° or more. The contact angle of the inner surface 52a of the hollow portion 52 is preferably 80° or less, and more preferably 70° or less.

### 2. Second Embodiment

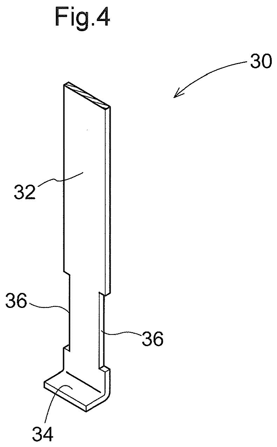

The following describes the holder 30 according to a second embodiment with reference to Fig. 4. In the present embodiment, the shape of the holder 30 is different from that in the first embodiment. The following mainly describes differences from the first embodiment. Note that points not described particularly are the same as those in the first embodiment. The pair of holders 30 each include a holder body 32 extending downward from the base plate 20, and a bent end 34 formed by bending a lower end portion of the holder body 32. In the present embodiment, the holder body 32 has recesses 36. Due to the recesses 36, the cell aggregate 15 retained by the pair of holders 30 can be aggregated generally. Because of this, at the time when the thickness of the cell aggregate 15 around the recess 36 increases and the cell aggregate 15 beats, stress can hardly concentrate on part of the cell aggregate 15. As a result, the cell aggregate 15 is hard to be damaged. More specifically, as illustrated in Fig. 4, the pair of holders 30 are a pair of film bodies facing each other, and the bent end 34 is formed by bending a lower end portion of the holder body 32 as a film body. The holder body 32 has the recesses 36 on the opposite side surfaces of the holder body 32 in its width direction. By placing the cell aggregate 15 on respective bent ends 34 of the pair of holders 30, the cell aggregate 15 can be retained stably by such a pair of bent ends 34. The pair of bent ends 34 may be formed by bending respective lower end portions of corresponding holder bodies 32 in the opposite directions or may be formed by bending respective lower end portions of corresponding holder bodies 32 to approach each other. Preferably, the cell aggregate 15 is placed on the pair of bent ends 34 and cultured, so that the cell aggregate 15 after the culture wraps the pair of bent ends 34. Hereby, the cell aggregate 15 can be more stably retained.

### 3. Third Embodiment

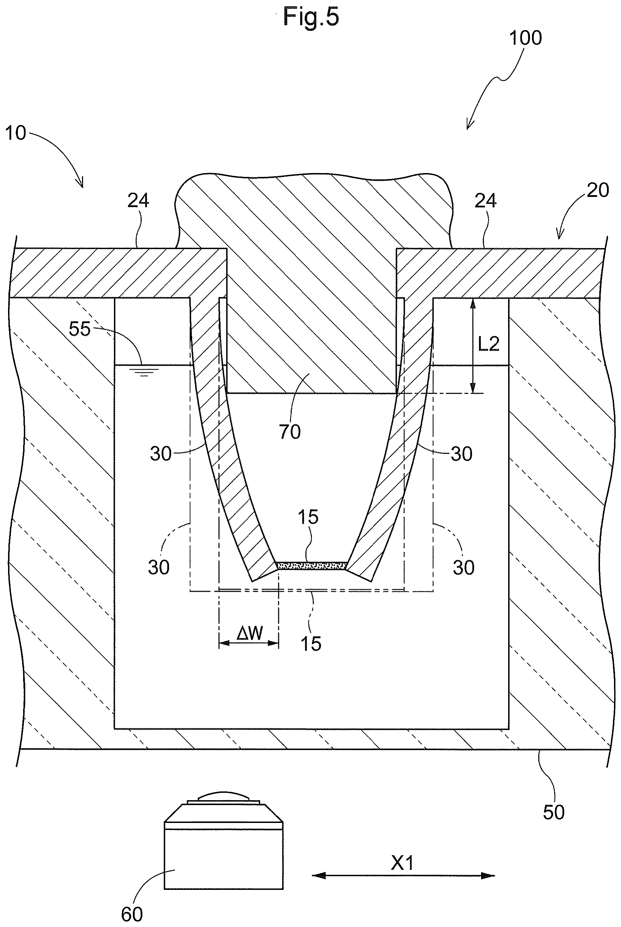

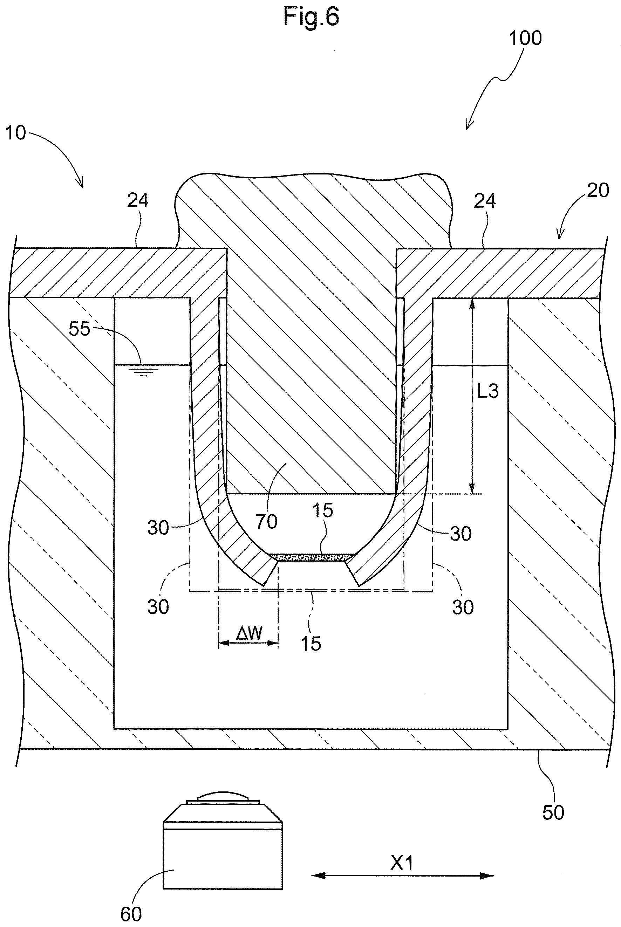

The following describes the cell characteristic detection set 100 according to a third embodiment with reference to Figs. 5 and 6. Fig. 5 is a sectional view of the cell characteristic detection set 100 according to the third embodiment and illustrates a contact member 70 attached to the base plate 20. Fig. 6 is a sectional view of the cell characteristic detection set 100 according to the third embodiment and illustrates a contact member 70 attached to the base plate 20, the contact member 70 being longer than the contact member 70 illustrated in Fig. 5 in length from the base plate 20.

The cell characteristic detection set 100 according to the third embodiment is different from the cell characteristic detection sets 100 according to the first and second embodiments in that the cell characteristic detection device 10 further includes the contact member 70. The following mainly describes differences from the first and second embodiments. Note that points not described particularly are the same as those in the first and second embodiments.

As illustrated in Fig. 5, the contact member 70 is provided for a region adjacent to the base plate 20 between the pair of holders 30 so that the contact member 70 can abut with the pair of holders 30 from inside in response to contraction of the cell aggregate 15. The region adjacent to the base plate 20 is a region between the base plate 20 and the cell aggregate 15. The distance between each holder 30 and the contact member 70 is determined such that each holder 30 deforming due to contraction of the cell aggregate 15 abuts with the contact member 70. The contact member 70 illustrated in Fig. 5 is attached to the base plate 20 in such a manner as to be suspended downward from the base plate 20 toward the cell aggregate 15, in a region between the pair of holders 30. Note that the contact member 70 may be attached to a portion other than the base plate 20, provided that the contact member 70 is provided for the region adjacent to the base plate 20. One contact member 70 may not abut with both the pair of holders 30. For example, a pair of contact members 70 may be provided such that the contact members 70 may abut with different holders 30.

In response to contraction of the cell aggregate 15, the pair of holders 30 elastically deform from respective starting points at portions close to the base plate 20, such that respective lower end portions of the holders 30 approach each other. Due to the deformation of the holders 30 by the contraction of the cell aggregate 15, the contact member 70 abuts with the pair of holders 30. Since the pair of holders 30 deforming by the contraction of the cell aggregate 15 abut with the contact member 70, the contact member 70 restrains the deformation of the pair of holders 30. Accordingly, the contact member 70 resists the contraction of the cell aggregate 15. As a result, in a case where a normal strain is used, for example, a resistance is given to the cell aggregate 15 to contract, so that the cell aggregate 15 strongly grows. In the meantime, in a case where a pathological strain is used, when a resistance is given to the cell aggregate 15, the cell aggregate 15 weakly grows.

It is preferable that the contact member 70 be attachable to and detachable from the base plate 20. With this configuration, in a case where the cell aggregate 15 to contract does not require any resistance, the contact member 70 is detached, so that no resistance is given to the cell aggregate 15 to contract. Accordingly, presence or absence of a resistance to the cell aggregate 15 to contract is switched depending on the cultural degree of the cell aggregate 15.

It is preferable that there are a plurality of contact members 70 attachable to and detachable from the base plate 20. In addition, it is preferable that the plurality of contact members 70 have different lengths from the base plate 20. The length of the contact member 70 from the base plate 20 is a length from a bottom surface of the bottom plate portion 24 of the base plate 20 to a lower end portion of the contact member 70. As the length from the base plate 20 is longer, the holder 30 abuts with the contact member 70 at a position farther from the base plate 20 (that is, a position closer to the cell aggregate 15) at the time when the cell aggregate 15 concentrates. Accordingly, the resistance that the contact member 70 gives to the cell aggregate 15 to contract varies depending on the length of the contact member 70 and increases as the length of the contact member 70 is longer. As a result, by attaching, to the base plate 20, the contact member 70 having a length corresponding to the cultural situation of the cell aggregate 15, a resistance with an appropriate magnitude corresponding to the cultural situation of cell aggregate 15 can be given to the cell aggregate 15 to contract.

More specifically, Fig. 6 illustrates the contact member 70 having a length L3 from the base plate 20, which length L3 is longer than a length L2 of the contact member 70 in Fig. 5 from the base plate 20. As illustrated in Fig. 6, in a case where the length L3 of the contact member 70 from the base plate 20 is longer than the length L2 of the contact member 70 from the base plate 20 as illustrated in Fig. 5, when the cell aggregate 15 contracts, the contact member 70 illustrated in Fig. 6 abuts with each holder 30 at a position closer to the cell aggregate 15 than the contact member 70 illustrated in Fig. 5. Accordingly, the contact member 70 illustrated in Fig. 6 gives a larger resistance to the cell aggregate 15 to contract than the contact member 70 illustrated in Fig. 5.

At the time of culturing, the cell aggregate 15 grows more strongly or more weakly in a shorter time, for example, by gradually raising a resistance to be given when the cell aggregate 15 contracts. Accordingly, at the time when the cell aggregate 15 is cultured, a strong cell aggregate 15 or a weak cell aggregate 15 can be cultured efficiently by changing the contact members 70 sequentially from the contact member 70 having a shorter length from the base plate 20 to the contact member 70 having a longer length from the base plate 20.

### 4. Other Embodiments

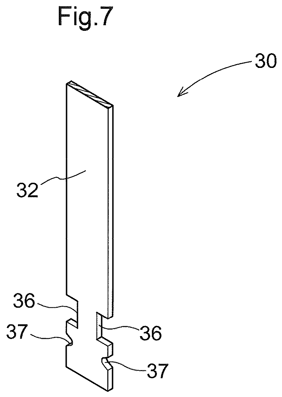

(1) The first embodiment (see Fig. 3) has described, as an example, the configuration in which the displacement amount ΔW of the holder 30 is measured by the optical microscope 60 from below the transparent or semitransparent container 50. However, the present invention is not limited to such an example, and the displacement amount ΔW may be measured laterally from the transparent container 50 by the optical microscope 60, for example. The measuring apparatus for detecting the displacement amount ΔW may be a measuring apparatus other than the optical microscope, for example. The displacement amount ΔW of the holder 30 may be detectable from outside by coupling a strain gauge to the holder 30, for example. The container 50 may not be a container through which light is transmissible, provided that the cell characteristic detection set 100 should be configured such that the displacement amount ΔW is detectable from outside.
(2) The first embodiment (see Fig. 1) has described, as an example, the configuration in which the pair of holders 30 are a pair of film bodies facing each other. However, the present invention is not limited to such an example, and the shape of the holder 30 may be a circular column shape or a square column shape, for example.
(3) The second embodiment (see Fig. 4) has described the holder 30 including the recesses 36 and the bent end 34. However, as illustrated in Fig. 7, the holder 30 may include the holder body 32 formed to have a flat surface, and notches 37 formed in the holder body 32. The notches 37 are formed on respective lateral surfaces of the holder body 32 in its width direction. In this configuration, a portion of the holder body 32 which portion is closer to its distal end than the notches 37 is easily bent, and a bending portion corresponding to the bent end 34 described in the second embodiment can be formed easily subsequently. By bending the portion of the holder body 32 which portion is closer to the distal end than the notches 37, the cell aggregate 15 is retained by the portion thus bent. Preferably, in the holder bodies 32 facing each other, their respective portions closer to respective distal ends than the notches 37 are bent perpendicularly to approach each other. Hereby, similarly to the second embodiment, the cell aggregate 15 can be retained stably. Here, it is preferable that the notches 37 be formed in the holder body 32 at a position away from the distal end of the holder body 32 by 0.5 mm to 2.0 mm.

The holder 30 illustrated in Fig. 7 includes the recesses 36 and the notches 37. Similarly to the recesses 36 in Fig. 4, the recesses 36 illustrated in Fig. 7 are formed on the opposite lateral surfaces of the holder body 32 in the width direction. In the meantime, the length of the recess 36 illustrated in Fig. 7 in its longitudinal direction is shorter than the length of the recess 36 illustrated in Fig. 4 in its longitudinal direction. The notches 37 are formed by cutting the opposite lateral surfaces of the holder body 32 in the width direction, at respective positions closer to the distal end of the holder body 32 than the recesses 36, in such a manner that the notches 37 have a triangular shape. Note that the positions, the sizes, the shapes, and the like of the recesses 36 and the notches 37 may be determined appropriately according to the type or the like of the cell aggregate 15.
(4) The first embodiment (see Fig. 1) has described, as an example, the configuration in which the cell aggregate 15 is a skeletal muscle cell aggregate, a cardiac muscle cell aggregate, or a smooth muscle cell aggregate, which is manufactured by inducing differentiation of induced pluripotent stem cells (iPS cells). However, the present invention is not limited to such an example, and the cell aggregate 15 may be a muscle cell aggregate, an adipocyte aggregate, a bone cell aggregate, a nerve cell aggregate, an epithelial cell aggregate, a cartilage cell aggregate, or tendinous tissue, which is manufactured by inducing differentiation of embryonic stem cells (ES cells), nuclear transfer embryonic stem cells (ntES cells), somatic stem cells, cord blood stem cells, and the like, for example. The cell aggregate 15 may be manufactured by a method other than the induced differentiation, for example.
(5) Note that the configurations disclosed in the above embodiments can be applied in combination with the configurations disclosed in other embodiments as long as no inconsistency occurs. In terms of other configurations, the embodiments disclosed in the present specification are also just examples in all respects. Accordingly, various modifications can be made appropriately without departing from the gist of this disclosure.

### Industrial Applicability

The technology according to this disclosure is applicable to a cell characteristic detection device for detecting contractability of a cardiac muscle cell aggregate, for example.

### Description of Reference Numerals

10: cell characteristic detection device
15: cell aggregate
20: base plate
30: holder
32: holder body
34: bent end
50: container
52a: inner surface
70: contact member
ΔW: displacement amount

## Claims

1. A cell characteristic detection device, comprising:
a base plate; and
a pair of holders made of resin, suspended from the base plate, and elastically deformable,
the cell characteristic detection device configured to allow an external device to detect an amount of displacement of at least one of the holders while the pair of holders are holding a cell aggregate therebetween.

2. The cell characteristic detection device according to claim 1, wherein
the pair of holders are a pair of film bodies facing each other.

3. The cell characteristic detection device according to claim 2, wherein
the pair of film bodies are made of a resin material having a Young's modulus of 100 MPa or more but 4500 MPa or less and each have a thickness of 5 µm or more but 400 µm or less.

4. The cell characteristic detection device according to claim 1, wherein
the pair of holders each include a holder body extending downward from the base plate, and a bent end in a form of a bend at a lower end of the holder body.

5. The cell characteristic detection device according to claim 1, wherein
the pair of holders are made of polystyrene-based resin, polypropylene-based resin, or polyethylene-based resin.

6. The cell characteristic detection device according to claim 1, wherein
the cell aggregate is a skeletal muscle cell aggregate, a cardiac muscle cell aggregate, or a smooth muscle cell aggregate.

7. The cell characteristic detection device according to claim 1, further comprising:
at least one contact member provided between the pair of holders and toward the base plate, the at least one contact member being configured to come into contact with the pair of holders from inside in response to contraction of the cell aggregate.

8. The cell characteristic detection device according to claim 7, wherein
the at least one contact member includes a plurality of contact members extending from the base plate over respective distances different from each other, the plurality of contact members being each attachable to and detachable from the base plate.

9. A cell characteristic detection set, comprising:
the cell characteristic detection device according to any one of claims 1 to 8; and
a container having an inner surface subjected to a cell non-specific adsorption inhibition treatment.
